# EUROPEAN PATENT APPLICATION

(11) **EP 3 616 531 A1**
(43) Date of publication of application: **04.03.2020**
(21) Application number: 18792158.0
(22) Date of filing: 27.04.2018
(51) Int. Cl.: A23L 5/00, A23K 20/158, A23K 20/163, A23K 20/179, A23L 5/44, A61K 8/06, A61K 8/60, A61K 8/73, A61Q 1/00, A61Q 19/00, B01F 17/56

(54) **EMULSION COMPOSITION**

(30) Priority: 28.04.2017 JP 2017089770
(71) Applicant: San-Ei Gen F.F.I., INC., Toyonaka-shi, Osaka 561-8588 (JP)
(72) Inventor: ITO,Takaaki, Toyonaka-shi Osaka 561-8588 (JP); KINOSHITA,Shunsuke, Toyonaka-shi Osaka 561-8588 (JP); MARUYAMA,Ryo, Toyonaka-shi Osaka 561-8588 (JP)
(74) Representative: Cabinet Beau de Loménie
(86) International application number: PCT/JP2018/017285
(87) International publication number: WO 2018/199315

(57) **Abstract**

The present invention provides an emulsion composition having excellent emulsion stability. Provided is an emulsion composition containing water, an oily component, gum ghatti, and saponin, in which a content of the saponin is 0.05 to 30 parts by mass based on 100 parts by mass of the gum ghatti.

## Description

### TECHNICAL FIELD

The present invention relates to an emulsion composition.

### BACKGROUND ART

Conventionally, an emulsion composition has been used which is obtained by emulsifying an oily component such as an oil-soluble pigment, an oil-soluble fragrance, or an oil-soluble physiologically active substance in order to disperse or dissolve the oily component in an aqueous medium.

For example, in Patent Document 1, an emulsion composition comprising a carotenoid as an oil-soluble pigment, a water-soluble emulsifier, tocopherol and lecithin is proposed. In Patent Document 2, a composition comprising gum ghatti, carotenoid, and an oil is proposed.

### PRIOR ART DOCUMENT

### PATENT DOCUMENTS

Patent Document 1: JP-A-2008-13751
Patent Document 2: JP-T-2011-521658

### SUMMARY OF THE INVENTION

### PROBLEMS TO BE SOLVED BY THE INVENTION

An object of the present invention is to provide an emulsion composition having improved emulsion stability.

### MEANS FOR SOLVING THE PROBLEMS

The inventors have researched intensively for an emulsion composition including gum ghatti, and found that an emulsion composition containing a carotenoid pigment as the oily component has a trend of coarsening of emulsion particles, and that the trend is a specific behavior showed when gum ghatti is used. Then, the inventors have further researched intensively, and found that an emulsion composition having excellent emulsion stability can be provided by containing gum ghatti and a certain amount of saponin to gum ghatti even if the composition contains a pigment such as a carotenoid pigment. This finding has led to the completion of the present invention.

The present invention includes the following aspects.

### Item 1.

An emulsion composition comprising water, an oily component, gum ghatti, and saponin, wherein a content of the saponin is from 0.05 to 30 parts by mass based on 100 parts by mass of the gum ghatti.

### Item 2.

The emulsion composition according to item 1, wherein the content of the saponin is from 0.01 to 10 parts by mass based on 100 parts by mass of the oily component.

### Item 3.

The emulsion composition according to item 1 or 2, wherein the oily component contains at least one kind selected from the group consisting of an oil-soluble pigment, an oil-soluble fragrance, an oil-soluble physiologically active substance, and an oily solvent.

### Item 4.

The emulsion composition according to any one of items 1 to 3, wherein the oily component contains a carotenoid pigment.

### Item 5.

The emulsion composition according to any one of items 1 to 3, wherein the oily component contains an oil-soluble fragrance.

### Item 6.

The emulsion composition according to any one of items 1 to 5, wherein emulsion particles in the emulsion composition have a median size of not more than 4 µm.

### Item 7.

The emulsion composition according to any one of items 1 to 6, wherein the emulsion composition has a liquid, powder, granule, or tablet form.

### Item 8.

A method for producing an emulsion composition, comprising:
preparing a mixed liquid containing water, an oily component, gum ghatti, and saponin, where a content of the saponin is from 0.05 to 30 parts by mass based on 100 parts by mass of the gum ghatti; and
homogenizing the mixed liquid.

### Item 9.

A method for producing a composition having a powder, granule, or tablet form, the method comprising the step of pulverizing the emulsion composition according to item 8.

### Item 10.

A method for producing a composition selected from the group consisting of a food and beverage, a cosmetic, a pharmaceutical product, a quasi-pharmaceutical product, a sanitary daily good, or an animal feed, the method comprising dissolving or dispersing the emulsion composition according to any one of items 1 to 7, or the composition having a powder, granule, or tablet form according to item 9 in an aqueous solvent.

### Item 11.

A food and beverage, a cosmetic, a pharmaceutical product, a quasi-pharmaceutical product, a sanitary daily good, or an animal feed, comprising the emulsion composition according to any one of items 1 to 7, or the composition having a powder, granule, or tablet form according to item 9.

### EFFECT OF THE INVENTION

According to the present invention, the emulsion composition having excellent emulsion stability is provided.

### BRIEF DESCRIPTION OF THE DRAWINGS

Fig. 1 is a photograph showing the colored result of crab-flavor steamed fish paste with the emulsion composition in Experimental Example 5.
Fig. 2 is a photograph showing the colored result of Bibimbap fried rice with the emulsion composition in Experimental Example 6.
Fig. 3 is a photograph showing the colored result of Kimchi with the emulsion composition in Experimental Example 7.
Fig. 4 is a photograph showing the colored result of Chinese steamed bun with the emulsion composition in Experimental Example 8.

### MODE FOR CARRYING OUT THE INVENTION

The present invention relates to an emulsion composition. Hereinafter, embodiments of the present invention will be described in detail.

As used herein, the term "include" is used to intend to include the term "essentially consist of" and the term "consist of".

### 1. Emulsion composition

The emulsion composition of the present invention can be suitably an oil-in-water type emulsion composition.

Stated more descriptively, the emulsion composition of the present invention can suitably include:
an aqueous phase which is a continuous phase containing water as a medium; and
an oil phase having a particle form containing an oil-soluble material and/or an oily solvent (which may be also referred to an oil-containing particle in the specification).

### Water

Examples of water used in the present invention include pure water, ion-exchanged water, and tap water.

Although the water content is not limited, for example, it can be not less than 5% by mass based on the total amount of the composition. The water content is preferably not less than 10% by mass, more preferably not less than 15% by mass, further preferably not less than 20% by mass, and particularly preferably not less than 25% by mass. In addition, although the water content is not limited, for example, the water content can be not more than 60% by mass based on the total amount of the composition. The water content is preferably not more than 55% by mass, more preferably not more than 50% by mass, further preferably not more than 45% by mass, even further preferably not more than 40% by mass, and particularly preferably not more than 35% by mass. Although the water content is not limited, for example, examples of the water content include an amount from 5 to 60% by mass, from 5 to 55% by mass, from 5 to 50% by mass, from 5 to 45% by mass, from 5 to 40% by mass, from 10 to 60% by mass, from 10 to 55% by mass, from 10 to 50% by mass, from 10 to 45% by mass, or from 10 to 40% by mass based on the total amount of the composition.

### Oily component

The oily component (that is, a component composing the oil phase) used in the present invention includes at least one kind selected from the group consisting of an oil-soluble material (the term includes a lipid-soluble material), and an oily solvent. As used herein, oily can mean a property in which the solubility at 20°C to one or both of n-hexane and ethyl acetate is not less than 10 g/L (preferably 50 g/L).

### Oil-soluble material

Examples of the oil-soluble material used in the present invention include an oil-soluble pigment, an oil-soluble fragrance, and an oil-soluble physiologically active substance.

### Oil-soluble pigment

The oil-soluble pigment used in the present invention (the term includes a lipid-soluble pigment) is not limited so long as it is an oil-soluble or lipid-soluble material containing a coloring component.

The oil-soluble pigment used in the present invention is preferably an edible pigment being capable of adding to a food and beverage or a pigment applicable to human body as a cosmetic.

Although the oil-soluble pigment is not limited as long as the pigment has the effect of the present invention, an example is a carotenoid pigment. The carotenoid pigment may be a natural pigment containing carotenoid. Examples of the carotenoid pigment include:
carotenes such as tomato pigment, Dunaliella carotene, carrot carotene, palm oil carotene, lycopene, phytoene, phytofluene, α-carotene, and β-carotene;
xanthophylls such as red pepper pigment (paprika pigment), Marigold pigment, Haematococcus algae pigment, capsanthin, capsorubin, lutein, zeaxanthin, astaxanthin, canthaxanthin, β-cryptoxanthin, violaxanthin, actinioerythritol, cucurbitaxanthin A, cryptocapsin, fucoxanthin, shrimp pigment, Krill pigment, crab pigment; and
apocarotenoids such as annatto pigment, bixin, β-8'-apo-carotenal (apocarotenal), β-12'-apo-carotenal, and derivatives thereof (esters with lower or higher alcohol), crocetin, and mycorradicin.

In addition, curcuma pigment, curcumin, and chlorophyll and the like are included. These oil-soluble pigment may be used alone, or in combination of two or more substances described above.

### Oil-soluble fragrance

The oil-soluble fragrance used in the present invention (the term includes a lipid-soluble fragrance) is not limited so long as it is an oil-soluble or lipid-soluble material containing a fragrance component.

The oil-soluble fragrance used in the present invention is preferably an edible fragrance being capable of adding to a food and beverage, or a fragrance applicable to human body as a cosmetic.

Examples of the fragrance include:
extracts obtained by non-volatile solvent extraction, volatile solvent extraction, or supercritical extraction and the like from animal nature or botanical natural raw materials or the combination thereof;
essential oils obtained by steam distillation or squeezing method and the like; natural fragrances such as recovered flavors;
synthetic fragrances which are fragrances synthesized by chemical means; and
fragrance bases obtained by adding and/or dissolving these fragrances to an oil and fat and/or a solvent.

Examples of the natural fragrance include:
extracts such as absolute, essence, and oleoresin;
squeezing liquid obtained by cold pressing, and the like; and
extracts with an alcohol, or extracts with a mixed liquid of water and an alcohol (for these extracts, so called a tincture).

Specific examples of these fragrances can include:
citrus essential oils such as orange oil, lemon oil, grapefruit oil, lime oil, and mandarin oil;
flower essential oils such as lavender oil (or absolutes);
essential oils such as peppermint oil, spearmint oil, and cinnamon oil;
spice essential oils (or oleoresins) such as allspice, anise seed, basil, laurel, cardamom, celery, clove, garlic, ginger, mustard, onion, paprika, parsley, and black pepper;
synthetic fragrances such as limonene, linalool, geraniol, menthol, eugenol, and vanillin;
bean-based extracted oils such as coffee, cacao, vanilla, and roasted peanuts;
tea-based essentials such as black tea, green tee, and oolong tea; and
synthetic fragrance compounds.

Although the fragrance can be used alone, the fragrance is typically used as a combined fragrance by using two or more fragrances in any combination.

The "fragrance" as used herein is defined as the concept including the fragrance composed of a single compound and the combined fragrance described above.

### Oil-soluble physiologically active substance

The oil-soluble physiologically active substance used in the present invention (the term includes a lipid-soluble physiologically active substance) is not limited so long as it is an oil-soluble or lipid-soluble material useful for living body.

The oil-soluble physiologically active substance used in the present invention is preferably an edible material being capable of adding to a food and beverage or a material applicable to human body as a cosmetic.

Examples of the oil-soluble physiologically active substance include:
an oil-soluble pharmaceutical agent;
lipid-soluble vitamins such as liver oil, vitamin A (for example, retinol), vitamin A oil, vitamin D (for example, ergocalciferol, and cholecalciferol), vitamin B₂ butyric acid ester, ascorbic acid fatty acid ester, vitamin E (for example, tocopherol, tocotrienol, and tocopherol acetic acid ester), and vitamin K (for example, phylloquinone, and menaquinone);
plant essential oils such as limonene, linalool, nerol, citronellol, geraniol, citral, 1-menthol, eugenol, cinnamicaldehyde, anethole, perillaldehyde, vanillin, and γ-undecalactone;
resveratrol, oil-soluble polyphenols, glycosyl ceramide, sesamin, phosphatidylserine, co-enzyme Q10, ubiquinol, and α-lipoic acid;
omega-3 fatty acids such as α-linolenic acid, eicosapentaenoic acid, and docosahexaenoic acid;
omega-6 fatty acids such as linoleic acid, and gamma-linolenic acid; and
functional materials such as plant sterols.

Among them, suitable examples include:
lipid-soluble vitamins, co-enzyme Q10, and α-lipoic acid; and
omega-3 fatty acids such as α-linolenic acid, eicosapentaenoic acid, and docosahexaenoic acid.

These oil-soluble physiologically active substances may be used alone, or in combination of two or more substances described above.

### Oily solvent

The oily solvent used in the present invention can suitably be one usable as a solvent for the oil-soluble material, and more specifically one which is compatible with the oil-soluble material.

The oily solvent used in the present invention is preferably an edible material being capable of adding to a food and beverage or a material applicable to human body as a cosmetic.

Examples of the oily solvent used in the present invention include:
plant oils and fats such as rapeseed oil, corn oil, palm oil, soybean oil, olive oil, jojoba oil, palm oil, Elemi resin, and Mastic resin;
animal oils and fats such as beef tallow and lard; and
sucrose acetic acid isobutyrate ester (SAIB), rosins, dammer resins, ester gums, glycerin fatty acid esters, and triglycerides.

It may be used alone, or in combination of two or more substances described above.

Preferred examples of the oily solvent include plant oils and fats, sucrose acetic acid isobutyrate ester (SAIB), glycerin fatty acid ester, and triglycerides. More preferred include plant oils and fats, glycerin fatty acid ester, and triglycerides (more preferably medium-chain triglycerides (MCT)).

Although a content of the oily component in the emulsion composition of the present invention is not limited, for example, it may be from 0.1 to 40% by mass based on the total amount of the composition, preferably from 0.3 to 40% by mass, more preferably from 0.5 to 35% by mass, further preferably from 0.5 to 33% by mass, furthermore preferably from 0.8 to 33% by mass, particularly preferably from 1 to 30% by mass, and most preferably from 1 to 28% by mass.

Typically, increasing the content of the oily component tends to decrease the emulsion stability of the emulsion composition. However, according to the present invention, the emulsion composition having excellent emulsion stability can be provided even if the content of the oily component is, for example, not less than 5% by mass, not less than 6% by mass, not less than 7% by mass, not less than 8% by mass, not less than 9% by mass, not less than 10% by mass, not less than 11% by mass, not less than 12% by mass, not less than 13% by mass, not less than 14% by mass, not less than 15% by mass, not less than 16% by mass, not less than 17% by mass, not less than 18% by mass, not less than 19% by mass, not less than 20% by mass, not less than 21% by mass, not less than 22% by mass, not less than 23% by mass, not less than 24% by mass, or not less than 25% by mass based on the total amount of the composition.

When the oily component obtained by extracting with solvent from animals and plants or microorganisms is used as the oily component, emulsion particles of the prepared emulsion composition tend to coarsen and decrease the emulsion stability, compared to the composition using the oily component which is different from the above oily component. That is because the oily component obtained by extracting with solvent from animals and plants or microorganisms contains impurities derived from the raw material. For example, when the oily component containing a carotenoid pigment derived from natural raw material (hereinafter, referred as "natural carotenoid pigment") is used as the oily component, the emulsion particle size of the emulsion composition tends to coarsen and decrease the emulsion stability, compared to the emulsion composition containing a synthetic carotenoid pigment.

However, according to the present invention, the emulsion composition having excellent emulsion stability can be provided even if the composition contains the oily component obtained by extracting with solvent from animals and plants or microorganisms as the oily component.

### Acetone insolubles

The above trend is particularly remarkable when "acetone insolubles" in the emulsion composition is not less than 0.025% by mass, further not less than 0.04% by mass, furthermore not less than 0.06% by mass, and particularly not less than 0.1% by mass based on the total amount of the composition.

However, according to the present invention, the emulsion composition having excellent emulsion stability can be provided even if "acetone insolubles" content in the emulsion composition is the content described above.

The "acetone insolubles" content in the emulsion composition can be calculated by measuring the acetone insolubles content in the oily component, and multiplying it by a ratio of the oily component containing acetone insolubles in the emulsion composition.

For example, when the emulsion composition contains 10% by mass of the oily component having acetone insolubles content of 1% by mass, the acetone insolubles content in the emulsion composition can be calculated as 0.1% by mass.

The acetone insolubles content in the oily component can be measured as follows.

### [acetone solubles included in the oily component, measurement conditions]

The measurement is according to the measurement method of "4.3.1-₁₉₉₆ acetone insolubles" in "Standard methods for the analysis of fats, oils and related materials (2013 version)" established by Japan Oil Chemists' Society.

In the present invention, although the upper limit of "acetone insolubles" content in the emulsion composition is not particularly limited, examples of the content include 10% by mass, 9% by mass, 8% by mass, 7% by mass, 6% by mass, 5% by mass, 4% by mass, 3% by mass, and 2% by mass based on the total amount of the composition.

### Gum ghatti

Gum ghatti is a polysaccharide derived from a sap (secretion) of Anogeissus latifolia Wallich (Anogeissus Latifolia), and is a polysaccharide known as a thickening stabilizer (food additive). Gum ghatti used in the present invention is commercially available, and example includes "gum ghatti RD" manufactured by San-Ei Gen F.F.I., Inc.

Although the gum ghatti may be gum ghatti obtained by decreasing the molecular weight (small molecule gum ghatti), without limitation. Although the weight-average molecular weight of small molecule gum ghatti is not limited, for example, it may be from 0.020×10⁶ to 1.10×10⁶, preferably from 0.020×10⁶ to 0.90×10⁶, more preferably from 0.020×10⁶ to 0.60×10⁶, further preferably from 0.025×10⁶ to 0.50×10⁶, furthermore preferably from 0.030×10⁶ to 0.40×10⁶, particularly preferably from 0.030×10⁶ to 0.30×10⁶, and further particularly preferably from 0.040×10⁶ to 0.30×10⁶.

The molecular weight of gum ghatti and the distribution thereof were measured by the following method. [Method for measuring molecular weight and molecular weight distribution]

The molecular weight and molecular weight distribution are measured by GPC analysis in the following conditions.
Detector: RI
Mobile layer: 100 mM K₂SO₄
Flow rate: 1.0 ml/min
Temperature: 40°C
Column: TSKgel GMPWXL 30 cm (Guard PWXL)
Injection: 100 µl
Pullulan standard: Shodex STANDARD P-82

### Method for producing small molecule gum ghatti

The small molecule gum ghatti of the present invention can be produced by the producing method as described below, or the similar method thereof. The method for producing the small molecule gum ghatti of the present invention includes decreasing the molecular weight of gum ghatti which is a raw material. As the gum ghatti which is a raw material, commercially available gum ghatti can be used.

The weight-average molecular weight of commercially available gum ghatti is typically within a range of 1.1×10⁶ to 2×10⁶. The raw material gum ghatti is not particularly limited as long as the gum ghatti having a molecular weight of interest can be produced. It may originally include low molecular weight gum ghatti in a part thereof.

Although the method for decreasing the molecular weight in the producing method is not particularly limited, suitable example includes a method for decreasing the molecular weight in the presence of water, including one or more treatment method selected from the group consisting of thermolysis treatment, acidolysis treatment, and enzymolysis treatment.

The thermolysis treatment is performed by suitably selecting the conditions for obtaining gum ghatti having a desired weight-average molecular weight depending on the technical knowledge in the art.

Typically, higher treatment temperature tends to obtain gum ghatti having lower weight-average molecular weight.

More specifically, the treatment temperature of the thermolysis treatment may be within a range of 60 to 200°C, and preferably a range of 80 to 200°C.

Typically, longer treatment time tends to obtain gum ghatti having lower weight-average molecular weight.

More specifically, the treatment time of the thermolysis treatment may be within a range of 30 sec to 8 hours. Note that the time can be suitably selected depending on the treatment temperature of thermolysis treatment. The treatment temperature and the treatment time can be suitably selected, for example, when the treatment temperature is high, the treatment time is decreased.

The thermolysis treatment can be suitably performed at a pH condition of pH 5 or less, for example.

Examples of the acid used for the acidolysis treatment include citric acid (contains citric acid anhydride), phosphoric acid, phytic acid, malic acid, tartaric acid, hydrochloric acid, acetic acid, lactic acid, and ascorbic acid.

The acid can be used alone, or two or more can be used in combination.

Typically, higher treatment temperature tends to obtain gum ghatti having lower weight-average molecular weight.

The treatment temperature of the acidolysis treatment may be within a range of 60 to 200°C, for example.

Typically, longer treatment time tends to obtain gum ghatti having lower weight-average molecular weight.

The treatment time of the acidolysis treatment may be within a range of 30 sec to 8 hours.

The acidolysis treatment can be suitably performed at a pH condition of pH 4 or less, for example.

Examples of the enzyme which can be used for the enzymolysis treatment include cellulase; mannanase; pectinase; sucrase; hemicellulase; Cellrosin AC40, Cellrosin HC100, Cellrosin TP25, and Cellrosin GM5 (manufactured by HBI Enzymes Inc.); Sumizyme PX, and Sumizyme AG2-L (manufactured by SHINNIHON CHEMICALS Corporation); Macerozyme A (manufactured by Yakult Pharmaceutical Industry Co., Ltd.); and Macerating enzyme Y (manufactured by Yakult Pharmaceutical Industry Co., Ltd.).

The enzyme can be used alone, or two or more can be used in combination.

The treatment conditions for the enzyme treatment (for example, temperature, time, pH and additives) can be suitably selected depending on the enzyme used.

Although the gum ghatti content in the emulsion composition according to the present invention is not particularly limited, for example, it is not less than 0.5% by mass, preferably not less than 0.8% by mass, more preferably not less than 1% by mass, further preferably not less than 1.5% by mass, and furthermore preferably not less than 2% by mass based on the total amount of the composition. The upper limit of the gum ghatti in the emulsion composition of the present invention is not more than 20% by mass, for example. It is preferably not more than 15% by mass, more preferably not more than 13% by mass, further preferably not more than 12% by mass, furthermore preferably not more than 10% by mass, particularly preferably not more than 9% by mass, particularly preferably not more than 8% by mass, and most preferably not more than 7.5% by mass.

The gum ghatti content in the emulsion composition of the present invention can be, for example, from 0.5 to 20% by mass, from 0.5 to 15% by mass, from 0.5 to 13% by mass, from 0.8 to 20% by mass. It is preferably from 0.8 to 13% by mass, more preferably from 1 to 13% by mass, further preferably from 1 to 10% by mass, furthermore preferably from 1.5 to 9% by mass, particularly preferably from 1.5 to 8% by mass, more particularly preferably from 1.5 to 7% by mass, and most preferably from 2 to 7% by mass.

Further, the gum ghatti content in the emulsion composition of the present invention is, for example, from 1 to 1000 parts by mass and from 1 to 800 parts by mass, preferably from 3 to 1000 parts by mass, more preferably from 4 to 1000 parts by mass, further preferably from 5 to 800 parts by mass, furthermore preferably from 8 to 500 parts by mass, particularly preferably from 10 to 500 parts by mass, and most preferably from 10 to 400 parts by mass based on 100 parts by mass of the oily component.

Further, as one suitable aspect, when the content of the oily component in the emulsion composition is higher than 5% by mass, the gum ghatti content based on 100 parts by mass of the oily component is preferably from 3 to 150 parts by mass, more preferably from 4 to 100 parts by mass, further preferably from 5 to 80 parts by mass, furthermore preferably from 8 to 70 parts by mass, and particularly preferably from 10 to 50 parts by mass.

Further, as one suitable aspect, when the content of the oily component in the emulsion composition is not more than 5% by mass, the gum ghatti content based on 100 parts by mass of the oily component is, for example, from 15 to 1000 parts by mass, preferably from 20 to 1000 parts by mass, more preferably from 30 to 800 parts by mass, further preferably from 50 to 600 parts by mass, furthermore preferably from 50 to 500 parts by mass, particularly preferably from 60 to 500 parts by mass, and most preferably from 60 to 450 parts by mass.

### Saponin

Saponin is a generic name of glycoside containing sapogenin as the aglycone. In the present invention, a plant extract containing a large amount of saponin can be used as saponin. Examples of the plant extract include Quillaja extract, Yucca extract, Asian ginseng extract, soybean extract, Tea seeds extract, and Japanese pagoda tree extract.

A content of the saponin in the emulsion composition of the present invention is within a range of 0.05 to 30 parts by mass based on 100 parts by mass of the gum ghatti. If the content of the saponin based on 100 parts by mass of the gum ghatti is lower than 0.05 parts by mass, the emulsion composition tends to have insufficient emulsion stability and the coarsening of emulsion particles. Similarly, if the content of the saponin based on 100 parts by mass of the gum ghatti is higher than 30 parts by mass, the emulsion stability of the emulsion composition is decreased.

In the present invention, within the range of the content of the saponin described above, the content of the saponin can be adjusted depending on the content of the oily component in the emulsion composition.

For example, when the content of the oily component in the emulsion composition is from 0.1 to 5% by mass, the content of the saponin based on 100 parts by mass of the gum ghatti is preferably from 0.05 to 5 parts by mass, more preferably from 0.08 to 4 parts by mass, further preferably from 0.1 to 3 parts by mass, furthermore preferably from 0.1 to 2 parts by mass, particularly preferably from 0.15 to 1.8 parts by mass, more particularly preferably from 0.15 to 1.6 parts by mass, and most preferably from 0.15 to 1.2 parts by mass.

On the other hand, when the content of the oily component in the emulsion composition is higher than 5% by mass, the content of the saponin based on 100 parts by mass of the gum ghatti is, for example, from 1 to 30 parts by mass, preferably from 1.5 to 30 parts by mass, more preferably from 1.5 to 29 parts by mass, further preferably from 2 to 28 parts by mass, furthermore preferably from 2.5 to 28 parts by mass, particularly preferably from 2.8 to 25 parts by mass, more particularly preferably from 3 to 20 parts by mass, and most preferably from 4 to 18 parts by mass.

Although the content of the saponin in the emulsion composition of the present invention is not particularly limited, it is preferably within a range of 0.0005 to 2% by mass, and the content of the saponin can be suitably adjusted depending on the content of the oily component in the emulsion composition.

For example, when the content of the oily component in the emulsion composition is from 0.1 to 5% by mass, the content of the saponin in the emulsion composition is preferably from 0.0005 to 0.1% by mass, more preferably from 0.001 to 0.08% by mass, further preferably from 0.005 to 0.08% by mass, furthermore preferably from 0.008 to 0.07% by mass, and most preferably from 0.01 to 0.05% by mass.

Also, when the content of the oily component in the emulsion composition is higher than 5% by mass, the content of the saponin in the emulsion composition is preferably from 0.01 to 3% by mass, more preferably from 0.05 to 2.5% by mass, further preferably from 0.08 to 2% by mass, furthermore preferably from 0.08 to 1.5% by mass, and most preferably from 0.1 to 1.2% by mass.

Although the content of the saponin in the emulsion composition of the present invention is also not particularly limited, the preferred saponin content is within a range of 0.01 to 10 parts by mass based on 100 parts by mass of the oily component. The content of the saponin can be suitably adjusted depending on the content of the oily component in the emulsion composition. The content of the saponin based on 100 parts by mass of the oily component is preferably from 0.1 to 10 parts by mass, more preferably from 0.2 to 8 parts by mass, furthermore preferably from 0.25 to 7 parts by mass, particularly preferably from 0.3 to 6 parts by mass, more particularly preferably from 0.3 to 5 parts by mass, and most preferably from 0.3 to 4 parts by mass.

### Emulsion particle size

The median size of the emulsion particles in the emulsion composition (by volume) can be, without limitation, not more than 4 µm, preferably not more than 2.5 µm, more preferably not more than 2 µm, further preferably not more than 1.5 µm, and particularly preferably not more than 1 µm. The median size (by volume) is, without limitation, preferably obtained by mixing an aqueous phase containing water, gum ghatti and saponin, with an oil phase containing the oily component by the condition such as mixing at 3,000 rpm for 3 minutes.

The lower limit of the median size (by volume) is not particularly limited, and can be suitably adjusted depending on the emulsion composition of interest. The lower limit of the median size (by volume) is not less than 0.05 µm, for example.

In the specification, the median size of the emulsion particles in the emulsion composition (by volume) is measured as follows. A cumulative particle size distribution curve by volume is obtained by measuring the particle size distribution of the sample using a laser diffraction/scattering particle size distribution measuring apparatus by Microtrac MT3000EX-II (MicrotracBEL Corp.). The median size of the emulsion particles in the emulsion composition is a volume accumulated particle size (D50) in which the cumulative particle size is 50 % by volume in the particle distribution measured by particle size viewing from the side of finer particles on 50% cumulation in the obtained cumulative particle size distribution curve. The median size of the emulsion particles in the emulsion composition (by volume) is described in detail in the test item of the Examples described below.

Since the emulsion composition of the present invention has excellent emulsion stability, the median size of the emulsion particles can be further decreased by homogenizing treatment using a high pressure homogenizer, a homodisper, a homomixer, a polytron based agitator, a colloid mill, and a nanomizer and the like. Although the homogenizing using a high-pressure homogenizer is not limited, a homogenizer (Homogenizer 15MR-8TA, manufactured by MANTON-GAULIN) can be used in a condition of 350 kg/cm², four times.

The median size of the emulsion particles in the emulsion composition (by volume) after homogenizing treatment can be, without limitation, not more than 2.5 µm, preferably not more than 2 µm, more preferably not more than 1.8 µm, further preferably not more than 1.6 µm, furthermore preferably not more than 1.3 µm, particularly preferably not more than 1 µm, and most preferably not more than 0.8 µm.

The frequency of the emulsion particles having a particle size of not less than 3.6 µm in the emulsion composition after homogenizing treatment can be, without limitation, not more than 60%, preferably not more than 40%, more preferably not more than 15%, further preferably not more than 10%, furthermore preferably not more than 8%, furthermore preferably not more than 5%, particularly preferably not more than 3%, and most preferably not more than 2%. Without limitation, since the emulsion composition of the present invention has excellent prolonged storage stability, the frequency of the emulsion particles having a particle size of not less than 3.6 µm in the emulsion composition can be preferably maintained even if the composition is stored at 60°C for 7 days.

The frequency of the emulsion particles having a particle size of not less than 1.3 µm in the emulsion composition after homogenizing treatment can be, without limitation, not more than 60%, preferably not more than 40%, more preferably not more than 30%, further preferably not more than 15%, furthermore preferably not more than 10%, particularly preferably not more than 8%, more particularly preferably not more than 5%, even further preferably not more than 3%, and most preferably not more than 2%. Without limitation, since the emulsion composition of the present invention has excellent prolonged storage stability, the frequency of the emulsion particles having a particle size of not less than 1.3 µm in the emulsion composition can be preferably maintained even if the composition is stored at 60°C for 7 days.

When the emulsion composition having the acetone insolubles content of not less than 0.025% by mass in the emulsion composition is prepared, or when the natural carotenoid pigment is used as the oily component, the emulsion composition having a median size of not more than 2 µm is difficult to be prepared. However, according to the present invention, even if the composition is in the above case, the emulsion composition in which the median size of the emulsion particles in the emulsion composition is, for example, not more than 4 µm, preferably not more than 2.5 µm, more preferably not more than 2 µm, further preferably not more than 1.5 µm, furthermore preferably not more than 1.3 µm, and particularly preferably not more than 1 µm can be provided.

Further, even if the content of the oily component in the emulsion composition is higher than 5% by mass, the emulsion composition containing emulsion particles having the median size described above can be prepared.

In this case, although the lower limit of the median size in the emulsion composition is not particularly limited, it can be not less than 0.3 µm, for example.

Further, according to the present invention, the emulsion composition having smaller median size (by volume) of the emulsion particles, for example, the emulsion composition in which the median size is preferably less than 0.3 µm, more preferably not more than 0.25 µm, and further preferably not more than 0.2 µm can be prepared.

Conventionally, when the emulsion composition using gum ghatti is dissolved or dispersed in the aqueous solvent, the turbidity may be provided in the composition. However, according to one embodiment of the present invention, a transparent-type emulsion composition that dissolves or disperses in the aqueous medium transparently can be provided. The transparent-type emulsion composition can be prepared by changing the content of the oily component to not more than 10% by mass, and preferably not more than 5% by mass.

In this case, although the lower limit of the median size in the emulsion composition is not particularly limited, it can be not less than 0.05 µm, for example.

### Polyalcohol

The emulsion composition of the present invention preferably may contain a polyalcohol. Thereby, the storage stability of the emulsion composition can be improved.

Examples of the polyalcohol which can be used in the present invention include glycerin, diglycerin, triglycerin, polyglycerin, propyleneglycol, dipropyleneglycol, 1,3-butyleneglycol, ethyleneglycol, polyethyleneglycol, sorbitol (D-sorbitol), xylitol, maltitol, erythritol, mannitol, xylose, glucose, lactose, mannose, oligotose, high-fructose corn syrup, and sucrose.

The polyalcohol may be used alone, or in combination of two or more substances described above.

Preferred polyalcohol in the present invention includes propyleneglycol, or glycerin, or combination thereof.

The polyalcohol content in the emulsion composition of the present invention is not particularly limited, and can be suitably adjusted depending on the emulsion composition of interest. The polyalcohol content in the emulsion composition is, for example, not less than 10% by mass, preferably not less than 15% by mass, more preferably not less than 20% by mass, and further preferably not less than 25% by mass based on the total amount of the composition. Although the upper limit of the polyalcohol content is not particularly limited, for example, it is not more than 60% by mass, preferably not more than 58% by mass, more preferably not more than 55% by mass, and further preferably not more than 50% by mass based on the total amount of the composition. Therefore, the polyalcohol content in the emulsion composition is, for example, from 10 to 60% by mass, preferably from 15 to 58% by mass, more preferably from 20 to 55% by mass, further preferably from 20 to 50% by mass, and most preferably from 25 to 50% by mass based on the total amount of the composition.

If the emulsion composition contains the polyalcohol, the emulsion particles may be coarsened. However, according to the present invention, the emulsion composition containing the emulsion particles having a median size within the range described above can be provided.

### pH

The pH of the emulsion composition of the present invention is suitably adjusted depending on the type of the compounded component and the content, and the formulation, and is not limited. For example, the pH can be within a range of 2 to 8, 2.5 to 7.5, or 3 to 7. Further, when the emulsion composition of the present invention is a liquid form, the pH is desirably within a range of 2 to 4, and preferably 2.5 to 3.5. For adjusting the pH of the emulsion composition to the range described above, an organic acid and/or inorganic acid can be used, if needed. The type of the organic acid and/or the inorganic acid is not particularly limited.

Examples of the organic acid and/or the inorganic acid include citric acid, phytic acid, ascorbic acid, phosphoric acid, lactic acid, adipic acid, gluconic acid, succinic acid, acetic acid, tartaric acid, fumaric acid, malic acid, and pyrophosphoric acid. The organic acid and/or the inorganic acid may be used alone, or in combination of two or more substances described above.

Preferred organic acid and/or inorganic acid in the present invention is one or more selected from the group consisting of citric acid, phytic acid, ascorbic acid, phosphoric acid, and lactic acid.

The emulsion composition of the present invention may include a water-soluble vitamin, a thickening stabilizer, an anti-oxidant, a chelating agent, or an oxidation inhibitor as other optioal ingredients within a range so that the effect of the present invention is not interfered. Further, the emulsion composition of the present invention may include ethanol within a range so that the effect of the present invention is not interfered.

The emulsion composition of the present invention may include an additional emulsifier within a range so that the effect of the present invention is not interfered. For example, preferred emulsifiers include lecithin (including lecithin, decomposed lecithin, and modified lecithin).

### Method for producing emulsion composition

The present invention also relates to a method for producing the emulsion composition described below.

A method for producing the emulsion composition, including:
preparing a mixed liquid containing water, an oily component, gum ghatti, and saponin, where a content of the saponin is from 0.05 to 30 parts by mass based on 100 parts by mass of the gum ghatti; and
homogenizing the mixed liquid.

In the producing method of the present invention, means, methods and conditions of the preparing step of the mixed liquid and the homogenizing treatment step are not particularly limited so long as the emulsion composition containing water, gum ghatti, saponin, and the oily component can be prepared. For example, when the emulsion composition can be prepared by mixing treatment of water, gum ghatti, saponin, and the oily component, the preparing step of the mixed liquid and the homogenizing treatment step can be one step (the same step).

The homogenizing treatment may be the homogenizing treatment using an emulsifying machine, including a homogenizer (for example, high-pressure homogenizer, a homodisper, a homomixer, a polytron based agitator, a colloid mill, and a nanomizer). The conditions for the homogenizing treatment may be suitably determined depending on the type of the emulsifying machine used and the like.

The formulation of the emulsion composition of the present invention is not particularly limited, the composition can be formulated into the liquid, the powder, the granule, or the tablet form depending on the type of the formulation such as a food and beverage, a cosmetic (including a cosmetic material), a pharmaceutical product, or a quasi-pharmaceutical product. For example, the particles of the emulsion composition of the present invention can make in the powder form by pulverizing means. The pulverizing means can perform by the normal method, for example, means such as spray drying or freeze drying can be performed. For pulverizing, a suitable carrier can be added. Further, the emulsion composition of the present invention may be in a liquid form obtained by re-dispersing the pulverized composition in the aqueous solvent.

From the viewpoint, the present invention relates to a method for producing the composition having a powder, granule, or tablet form, including the following aspect:
a step for producing a composition having a powder, granule, or tablet form, the method including:
preparing a mixed liquid containing water, an oily component, gum ghatti, and saponin, where a content of the saponin is from 0.05 to 30 parts by mass based on 100 parts by mass of the gum ghatti;
homogenizing the mixed liquid to prepare an emulsion composition; and
pulverizing the emulsion composition.

The resulting the composition having a powder, granule, or tablet form has advantages having excellent dispersity to the aqueous medium and emulsion stability after dispersing. The pulverizing method is not particularly limited, and can be performed according to the normal method. Examples of the pulverizing method include spray drying or freeze drying.

As one embodiment of the present invention, when the composition of the present invention is formulated into the powder, the granule, or the tablet form, the gum ghatti content in the composition can be, for example, from 0.01 to 30% by mass, from 0.5 to 30% by mass, from 1 to 30% by mass, from 3 to 30% by mass, from 5 to 30% by mass, from 7 to 30% by mass, from 10 to 30% by mass, from 13 to 30% by mass, from 15 to 30% by mass, from 1 to 20% by mass, from 3 to 20% by mass, from 5 to 20% by mass, from 7 to 20% by mass, from 10 to 20% by mass, from 13 to 20% by mass, from 15 to 20% by mass, from 1 to 15% by mass, from 3 to 15% by mass, from 5 to 15% by mass, from 7 to 15% by mass, from 10 to 15% by mass, or from 13 to 15% by mass based on the total amount of the composition.

As one embodiment of the present invention, when the composition of the present invention is formulated into the powder, the granule, or the tablet form, the content of the saponin in the composition can be, for example, from 0.0001 to 5% by mass, from 0.0005 to 5% by mass, from 0.001 to 5% by mass, from 0.005 to 5% by mass, from 0.01 to 5% by mass, from 0.03 to 5% by mass, from 0.05 to 5% by mass, from 0.1 to 5% by mass, from 0.2 to 5% by mass, from 0.01 to 3% by mass, from 0.03 to 3% by mass, from 0.05 to 3% by mass, from 0.1 to 3% by mass, from 0.2 to 3% by mass, from 0.01 to 1% by mass, from 0.03 to 1% by mass, from 0.05 to 1% by mass, from 0.1 to 1% by mass, from 0.2 to 1% by mass based on the total amount of the composition.

As one embodiment of the present invention, when the composition of the present invention is formulated into the powder, the granule, or the tablet form, the content of the oily component in the composition can be, for example, from 0.01 to 40% by mass, from 0.05 to 40% by mass, from 0.3 to 40% by mass, from 1 to 40% by mass, from 3 to 40% by mass, from 5 to 40% by mass, from 7 to 40% by mass, from 10 to 40% by mass, from 1 to 30% by mass, from 3 to 30% by mass, from 5 to 30% by mass, from 7 to 30% by mass, from 10 to 30% by mass, from 1 to 20% by mass, from 3 to 20% by mass, from 5 to 20% by mass, from 7 to 20% by mass, or from 10 to 20% by mass based on the total amount of the composition.

As one embodiment of the present invention, when the composition of the present invention is formulated into the powder, the granule, or the tablet form, the water content in the composition can be, for example, from 0.005 to 15% by mass, from 0.01 to 15% by mass, from 0.05 to 15% by mass, from 0.1 to 15% by mass, from 0.5 to 15% by mass, from 1 to 15% by mass, from 0.05 to 12% by mass, from 0.1 to 12% by mass, from 0.5 to 12% by mass, from 1 to 12% by mass, from 0.05 to 10% by mass, from 0.1 to 10% by mass, from 0.5 to 10% by mass, or from 1 to 10% by mass based on the total amount of the composition.

Without limitation, in the present invention, the emulsion composition can be produced without using an organic solvent. Examples of the organic solvent in which the emulsion composition can be produced without using include acetone, cyclohexane, 1-propanol, 2-propanol, and dichloromethane and the like.

### Applications

The emulsion composition of the present invention or the composition having a powder, granule, or tablet form can be used for various applications depending on the type of the oily component, for example. For example, if the oily component is a pigment, the composition can be applied as a pigment formulation. If the oily component is a fragrance, the composition can be applied as a fragrance formulation. If the oily component is a physiologically active substance, the composition can be applied as a physiologically active substance formulation. Also, the emulsion composition of the present invention or the composition having a powder, granule, or tablet form can be applied as a clouding agent (also referred as turbid agent, cloudy), which provides a suitable turbidity to the aqueous medium such as beverages.

Suitable aspect of the emulsion composition of the present invention or the composition having a powder, granule, or tablet form is a pigment formulation, and a more suitable aspect is a carotenoid pigment formulation.

Although the pigment content in the pigment formulation (includes emulsified carotenoid pigment) is not particularly limited, the color value (E^{10%}_{1cm}) is from 100 to 800, preferably from 200 to 700, and more preferably from 300 to 600.

As used herein, "color value (E^{10%}_{1cm})" is represented by a value (E^{10%}_{1cm}) obtained by converting the absorbance in the maximum adsorbing wavelength in the visible light area of the pigment-containing material such as the pigment formulation to the absorbance of 10% (w/v) solution, as understood by the skilled in the art of pigment.

In the present invention, the color value (E^{10%}_{1cm}) is a value determined according to "18. color value measuring method" in JAPAN'S SPECIFICATIONS AND STANDARDS FOR FOOD ADDITIVES, 9th version.

### Aqueous composition

The present invention relates to an aqueous composition containing the above emulsion composition, or the composition having a powder, granule, or tablet form.

Examples of the aqueous composition include, without limitation, a food and beverage, a cosmetic, a pharmaceutical product, a quasi-pharmaceutical product, a sanitary daily good, or an animal feed. Preferably, it can be a food and beverage, and more preferably a beverage.

The content of the emulsion composition of the present invention or the composition having a powder, granule, or tablet form in the aqueous composition may vary depending on the type of the composition and its application. For example, the content can be within a range of 0.001 to 5% by mass, or within a range of 0.01 to 1% by mass.

The present invention also relates to a food and beverage containing the above emulsion composition, or the composition having a powder, granule, or tablet form. The type of the food and beverage is not particularly limited. More specifically, examples of the food and beverage include:
beverages such as milk beverages, lactic acid bacteria beverages, carbonated beverages, fruit beverages (for example, fruit juice beverages, fruit juice-containing soft beverages, fruit juice-containing carbonated beverages, fruit pulp beverages), vegetable beverages, vegetable and fruit beverages, alcohol beverages such as liqueur, coffee beverages, powder beverages, sports beverages, and supplement beverages;
tea beverages such as black tea, green tee, and blend tea (note: beverages and tea beverages are included in "beverages");
puddings such as custard puddings, milk puddings, and fruit juice-containing puddings, jelly (for example, multilayered jelly), desserts such as Bavarian cream and yogurt;
frozen desserts such as ice creams, ice milks, lact ices, and sherbets;
gums such as chewing gums and bubble gums (for example, plate gums and sugar coated granule gums);
chocolates such as coated chocolates (for example, marble chocolates), flavored chocolates (for example, strawberry chocolates, blueberry chocolates, and melon chocolates);
candies such as hard candies (for example, bonbons, butter balls, and marbles), soft candies (for example, caramel, nougat, gummi candies, and marshmallows), sugar coated candies, drops, and taffies;
confectioneries such as cookies and biscuits;
soups such as consomme soups, and potage soups;
liquid seasonings such as separate dressings, non-oil dressings, ketchup, sauce (for example, sauce for roast meat, sauce for roast chicken, and sauce for broiled eel), other sauces, Chili oil;
other seasonings such as butter, and butter cream;
jams such as strawberry jam, blueberry jam, marmalade, apple jam, apricot jam, preserves, and syrups;
fruit liquors such as red wines;
fruits for processing preserved in syrup including cherry, apricot, apple, strawberry and peach;
agricultural processed product such as pickles (for example, Kimchi, red pickled ginger, pickled ume, pickled radish with pink colour, and yellow pickled radish);
daily dishes such as rolled omelette;
livestock meats such as ham, sausage, and roast pork;
marine processed products such as fish pastes, and fish egg products (for example, crab-flavor steamed fish paste, foods dried with mirin, fish salted and dried overnight, colored fish eggs, colored shrimps); and
breads, noodles (for example, non-fried noodles, Chinese noodles, dried noodles, and Cha soba), bun dough, grain processed foods such as rice (for example, Bibimbap fried rice).

Examples of the food and beverage include semifinished goods and intermediate goods of these products.

Examples of the "cosmetic" include skin lotions, lipsticks, sunscreen cosmetics, and make-up cosmetics.

Examples of the "pharmaceutical product" include various tablets, capsules, drink agents, troches, and gargles.

Examples of the "quasi-pharmaceutical product" include nutritional supplements, various supplements, dentifrice agents, mouth fresheners, odor preventing agents, hair growth stimulating agents, hair tonics, and moisturizers for skin.

Examples of the "sanitary daily good" include soaps, detergents, shampoos, rinses, hairdressings, dentifrice agents, and bath additives.

Examples of the "animal feed" include various pet foods such as cat foods and dog foods, and feeds for aquarium fish or cultured fish.

### Method for improving emulsion stability of emulsion composition (improving method of emulsion stability)

The present invention also relates to a method for improving the emulsion stability of the emulsion composition having the following aspect.

A method for improving the emulsion stability of the emulsion composition containing water, an oily component, and gum ghatti, including:
step to include saponin into the emulsion composition, in which
a content of the saponin is from 0.05 to 30 parts by mass based on 100 parts by mass of the gum ghatti.

The method may be the same as embodiments of the emulsion composition of the present invention and the method for producing thereof, or similar to them, and can be understood by reffering the method for producing the emulsion composition of the present invention.

The method for improving the emulsion stability of the emulsion composition in the present invention includes the aspect in which the step of including saponin in the emulsion composition is preferably performed before forming the emulsion particles.

The method for improving the emulsion stability of the emulsion composition of the present invention includes the following suitable aspects.

A method for improving the emulsion stability of the emulsion composition containing gum ghatti, the method including:
1) preparing a mixed liquid containing water, oily component, the gum ghatti, and the saponin; and
2) homogenizing the mixed liquid to prepare an emulsion composition.

### EXAMPLES

Hereinafter, although the present invention will be described with reference to Examples, the scope of the present invention is not limited to the Examples.

Hereinafter, unless otherwise specified, the value showing the composition in each table can represent % by mass.

### Materials

Paprika pigment (1): Paprika oleoresin, color value^{10%}_{1 cm} = 2,240, acetone insolubles 1.25% by mass
Paprika pigment (2): Paprika oleoresin, color value^{10%}_{1 cm} = 2,400, acetone insolubles 7.26% by mass
Marigold pigment: Marigold oleoresin, color value^{10%}_{1cm} = 4600, acetone insolubles 5.4% by mass
Haematococcus algae pigment: Haematococcus algae pigment, color value^{10%}_{1cm} = 2820, acetone insolubles 10.69% by mass
Extracted carotene (carrot carotene): carrot oleoresin, color value^{10%}_{1cm} = 250, acetone insolubles 9.37% by mass
Gum ghatti: weight-average molecular weight 1.18 million
Small molecule gum ghatti: weight-average molecular weight 0.15 million
Saponin: Quillajasaponin (saponin content 12.5% by mass, "Quillajanin C-50-MT(G)", MARUZEN PHARMACEUTICALS CO., LTD.), or Quillajasaponin (saponin content 7.5% by mass, "Quillaja extract S", MARUZEN PHARMACEUTICALS CO., LTD.)
Glycerin fatty acid ester: major fatty acid is oleic acid, HLB = 15, decaglycerin fatty acid ester

The small molecule gum ghatti was prepared according to the method described in WO 2018/062554. The weight-average molecular weight was measured by GPC analysis in the following conditions.
Detector: RI
Mobile phase: 100 mM K2SO4
Flow rate: 1.0 ml/min
Temperature: 40°C
Column: TSKgel GMPWXL 30 cm (Guard PWXL)
Injection: 100 µl
Pullulan standard: Shodex STANDARD P-82

### Emulsion stability test

The following test items were evaluated for emulsion compositions.
- D50 µm, or particle size D50: median size (µm)
- 3.6 µm_{↑} or 3.6 µ_{↑}: frequency of particle size of not less than 3.6 µm (%)
- 1.3 µm_{↑} or 1.3 µ_{↑}: frequency of particle size of not less than 1.3 µm (%)
- 0.1 %E (720 nm): turbidity of the sample (emulsion composition) in 0.1% water-diluted liquid at 720 nm

### (Emulsion particle size: median size, frequency of particle size)

For the frequency of the median size or the particle size, the particle size distribution of the emulsion composition was measured in a condition described below.

### <Conditions>

### Particle size distribution measuring apparatus: Microtrac MT3000EX-II (MicrotracBEL Corp.)

Measuring method: refractive index: 1.81, measurement range: 0.021 to 2000 µm, particle size distribution: by volume.

### (Turbidity)

One indicator for evaluating the emulsion particle size of the emulsion composition is a turbidity. For example, if the emulsion particle size is not more than 1 µm, smaller turbidity means smaller emulsion particle size.

In the test example, the stability of the emulsion composition was evaluated by measuring the turbidity of the emulsion composition.

The above "0.1% E (720 nm)" was measured by diluting each sample (emulsion composition) with ion-exchanged water to form 0.1% aqueous solution, and measuring the turbidity of the diluted solution at 720 nm in a condition described below.

### <Conditions>

Spectrophotometer: Spectrophotometer V-660DS, JASCO Corporation
Measurement conditions: quartz cell, 10 mm × 10 mm, absorbance (Abs)

### Experimental Example 1: Method for producing emulsion compositions (emulsion pigment compositions)

The emulsion compositions were produced according to the formulation shown in each Table.

The materials of the oil phase shown in Table 1 were mixed and heated to 100°C to prepare an oil phase. The oil phase was added to an aqueous phase (1), stirred at 3,000 rpm for 3 minutes to prepare a mixed liquid. An aqueous phase (2) was added to the mixed liquid, and stirred at 1,000 rpm for 1 minute. Next, the mixture was homogenized on a high-pressure homogenizer (Homogenizer 15MR-8TA, manufactured by MANTON-GAULIN) (condition: 350 kg/cm², four times).

The acetone insoluble content in the emulsion composition is 0.21% by mass. The pH of the emulsion composition prepared in Experimental Example 1 (Example and Comparative Example) was within a range of 2.5 to 3.5.

**[Table 1]**

| Formulation of emulsion composition, % by mass | | Example 1-1 | Example 1-2 | Example 1-3 | Comparative Examples 1-1 |
|---|---|---|---|---|---|
| Oil phase | Carotenoid pigment (paprika pigment (1), color value 2,240) | 17 | 17 | 17 | 17 |
| | Tocopherol | 0.5 | 0.5 | 0.5 | 0.5 |
| | Corn oil | 8 | 8 | 8 | 8 |
| | Total of oil phase | 25.5 | 25.5 | 25.5 | 25.5 |
| Aqueous phase (1) | 20% aqueous gum ghatti solution | 27.5 | 27.5 | 27.5 | 30 |
| | Quillajanin C-50-MT (G) the content of the saponin in parentheses | 1.7 (0.21) | 3.4 (0.43) | 6.8 (0.85) | - |
| Aqueous phase (2) | Glycerin | 19.5 | 19.5 | 19.5 | 19.5 |
| | Phytic acid | 0.5 | 0.5 | 0.5 | 0.5 |
| | Water | Balance (so as to be 100 in total) | | | |

An emulsion stability test was performed for the emulsion composition (the mixed liquid of the oil phase and the aqueous phase (1), and the composition after homogenizing). The test items "D50 µm", "3.6 µm_{↑}", and "1.3 µm_{↑}" were evaluated in the emulsion stability test. Results are shown in Table 2.

**[Table 2]**

| Emulsion composition | Mixed liquid | | Composition after homogenizing | | |
|---|---|---|---|---|---|
| | D50 µm | 3.6 µ_{↑} | D50 µm | 3.6 µ_{↑} | 1.3 µ_{↑} |
| Example 1-1 | 1.8 µm | 0.3% | 1.51 µm | 0% | 2.8% |
| Example 1-2 | 1.2 µm | 0% | 0.66 µm | 0% | 2.2% |
| Example 1-3 | 1.9 µm | 0.9% | 0.52 µm | 0% | 4.3% |
| Comparative Examples 1-1 | Unmeasurable | Unmeasurable | N.D | N.D | N.D |

For the composition of Comparative Example 1-1 using gum ghatti, the oil was separated during the preparation of the mixed liquid. Next, the composition was continued stirring. Since the oil phase and the aqueous phase could not be mixed thoroughly, the composition was determined that it was impossible to be emulsified.

On the other hand, from Examples 1-1 to 1-3 containing gum ghatti and a certain amount of saponin based on 100 parts by mass of gum ghatti, emulsion compositions having a median size of not more than 2 µm were obtained only by mixing the oil phase and the aqueous phase. By further homogenizing, the emulsion composition having smaller median size was prepared. Therefore, emulsion particles having excellent emulsion stability can be prepared by containing gum ghatti and a certain amount of saponin to gum ghatti.

According to the formulations shown in Table 3, the emulsion compositions were prepared similar as in the above examples, such as Example 1-1.

**[Table 3]**

| Formulation of emulsion composition, % by mass | | Comparative Examples 1-2 | Comparative Examples 1-3 | Comparative Examples 1-4 | Comparative Examples 1-5 |
|---|---|---|---|---|---|
| Oil phase | Carotenoid pigment (paprika pigment (1), color value 2,240) | 17 | 17 | 17 | 17 |
| | Tocopherol | 0.5 | 0.5 | 0.5 | 0.5 |
| | Corn oil | 8 | 8 | 8 | 8 |
| | Lysolecithin | - | - | 0.5 | - |
| | Glycerin fatty acid ester | - | - | - | 0.5 |
| | Total of oil phase | 25.5 | 25.5 | 26 | 26 |
| Aqueous phase (1) | 20% aqueous gum ghatti solution | 35 | 40 | 40 | 40 |
| Aqueous phase (2) | Glycerin | 10.9 | 10.7 | 10.7 | 10.7 |
| | Phytic acid | 0.5 | 0.5 | 0.5 | 0.5 |
| | Water | Balance (so as to be 100 in total) | | | |

An emulsion stability test was performed for the emulsion composition (the mixed liquid of the oil phase and the aqueous phase (1), and the composition after mixing the aqueous phase (2)). Results are shown in Table 4.

**[Table 4]**

| Emulsion composition | Mixed liquid | | After mixing aqueous phase (2) | |
|---|---|---|---|---|
| | D50 µm | 3.6 µ_{↑} | D50 µm | 3.6 µ_{↑} |
| Comparative Examples 1-2 | 2.1 µm | 40.5% | 36.4 µm | 82.4% |
| Comparative Examples 1-3 | 1.8 µm | 8.3% | 61.8 µm | 100% |
| Comparative Examples 1-4 | 2.0 µm | 2.4% | 34.4 µm | 87.6% |
| Comparative Examples 1-5 | 26.1 µm | 100% | 17.4 µm | 100% |

Comparative Examples 1-2 and 1-3 are examples in which the gum ghatti content in the emulsion composition is increased. Comparative Examples 1-4 and 1-5 are examples containing gum ghatti in addition to other emulsifiers (lysolecithin and glycerin fatty acid ester), respectively.

These have insufficient emulsifying of the mixed liquid or the composition after mixing the aqueous phase (2), and the composition having excellent emulsion stability could not be provided, as shown in Table 4.

### Experimental Example 2: Method for producing emulsion compositions (emulsion fragrance compositions)

The emulsion compositions were produced according to the formulation shown in each Table.

The materials of the oil phase shown in Table 5 were mixed to prepare an oil phase. The oil phase was added to an aqueous phase (1), stirred at 3,000 rpm for 3 minutes to prepare a mixed liquid. The mixed liquid was homogenized on a high-pressure homogenizer (Homogenizer 15MR-8TA, manufactured by MANTON-GAULIN) (condition: 500 kg/cm², five times). Next, an aqueous phase (2) was added to the mixed liquid, and stirred at 1,000 rpm for 1 minute.

The pH of the emulsion composition prepared in Experimental Example 2 (Example and Comparative Example) was within a range of 2 to 4, and the acetone insolubles content is less than 0.025% by mass.

**[Table 5]**

| Formulation of emulsion composition, % by mass | | Reference Example 2 | Example 2-1 | Example 2-2 | Example 2-3 | Example 2-4 | Example 2-5 |
|---|---|---|---|---|---|---|---|
| Oil phase | Lemon essential oil | 0.8 | 0.8 | 0.8 | 0.8 | 0.8 | 0.8 |
| | Lecithin | 0.1 | 0.1 | 0.1 | 0.1 | 0.1 | 0.1 |
| | Medium-chain triglycerides | 0.1 | 0.1 | 0.1 | 0.1 | 0.1 | 0.1 |
| | Tocopherol | 0.1 | 0.1 | 0.1 | 0.1 | 0.1 | 0.1 |
| | Total of oil phase | 1.1 | 1.1 | 1.1 | 1.1 | 1.1 | 1.1 |
| Aqueous phase (1) | 20% aqueous gum ghatti solution | 20 | 20 | 20 | 20 | 20 | 20 |
| | Quillaja extract S, the content of the saponin in parentheses | - | 0.1 (0.0075) | 0.2 (0.015) | 0.3 (0.0225) | 0.4 (0.03) | 0.5 (0.0375) |
| | Water | 25.9 | 25.8 | 25.7 | 25.6 | 25.5 | 25.4 |
| Aqueous phase (2) | Glycerin | 53 | 53 | 53 | 53 | 53 | 53 |

An emulsion stability test was performed for the emulsion composition (the composition after mixing the aqueous phase (2)). The test item "0.1% E (720 nm)" was evaluated in the emulsion stability test. Results are shown in Table 6.

**[Table 6]**

| | Reference Example 2 | Example 2-1 | Example 2-2 | Example 2-3 | Example 2-4 | Example 2-5 |
|---|---|---|---|---|---|---|
| 0.1%E(720nm) | 0.019 | 0.015 | 0.010 | 0.012 | 0.014 | 0.014 |

From Table 6, it was found that the turbidity of the emulsion composition is decreased by including a certain amount of saponin in addition to gum ghatti. When the emulsion particle size (median size) was measured for Example 2-1 having the biggest turbidity value among Examples, the value was less than 0.3 µm.

When the emulsion particle size of the emulsion composition is not more than 1 µm, the value of the turbidity 0.1% E (720 nm) is proportional to the emulsion particle size. Therefore, this results showed that the emulsion particle size of the emulsion composition can be decreased by including a certain amount of saponin in addition to gum ghatti.

### Experimental Example 3: Method for producing emulsion compositions (emulsion pigment compositions)

The emulsion compositions were produced according to the formulation shown in each Table.

The materials of the oil phase shown in Table 7 were mixed and heated to 80°C to prepare an oil phase. The oil phase was added to an aqueous phase (1), stirred at 3,000 rpm for 3 minutes to prepare a mixed liquid. An aqueous phase (2) was added to the mixed liquid, and stirred at 3,000 rpm for 1 minute. Next, the mixture was homogenized on a high-pressure homogenizer (Homogenizer 15MR-8TA, manufactured by MANTON-GAULIN) (condition: 560 kg/cm², four times).

**[Table 7]**

| Formulation of emulsion composition, % by mass | | Examples | | | | | | |
|---|---|---|---|---|---|---|---|---|
| | | 3-1 | 3-2 | 3-3 | 3-4 | 3-5 | 3-6 | 3-7 |
| Oil phase | Paprika pigment (2) | 19 | 9.5 | 4.7 | 1.9 | - | - | - |
| | Marigold pigment | - | - | - | - | 3 | - | - |
| | Haematococcus algae pigment | - | - | - | - | - | 7.5 | - |
| | Extracted carotene | - | - | - | - | - | - | 15.5 |
| | Tocopherol | 0.5 | - | 0.3 | 0.1 | 0.5 | 0.5 | 0.5 |
| | Safflower oil | 6 | - | - | - | - | - | - |
| | Medium-chain fatty acid triglyceride | - | 5.5 | 1 | 1 | 16.5 | 12 | 4 |
| | Total of oil phase | 25.5 | 15 | 6 | 3 | 20 | 20 | 20 |
| Aqueous phase (1) | 20% aqueous gum ghatti solution | 15 | 20 | 20 | 20 | 20 | 20 | 20 |
| | Quillajanin C-50-MT (G), the content of the saponin in the emulsion composition in parentheses | 6 (0.75) | 3 (0.375) | 1 (0.125) | 0.5 (0.0625) | 4 (0.5) | 4 (0.5) | 4 (0.5) |
| | Ion-exchanged water | 17.5 | 17.5 | 27.5 | 27.5 | 11.5 | 11.5 | 11.5 |
| Aqueous phase (2) | First lactic acid (50% aqueous solution) | 0.5 | 0.5 | 0.5 | 0.5 | 0.5 | 0.5 | 0.5 |
| | Glycerin | 35.5 | 44 | 45 | 48.5 | 44 | 44 | 44 |
| pH of emulsion composition | | 3.2 | 3.3 | 3.2 | 3.2 | 3.4 | 3.4 | 3.5 |
| Acetone insolubles content of the emulsion composition (%) | | 1.38 | 0.69 | 0.34 | 0.14 | 0.16 | 0.08 | 1.45 |

An emulsion stability test was performed for the emulsion composition (the mixed liquid of the oil phase and the aqueous phase (1), and the composition after homogenizing). The test items "D50 µm", "3.6 µm_{↑}", and "1.3 µm_{↑}" were evaluated in the emulsion stability test. Results are shown in Table 8.

**[Table 8]**

| Emulsion composition | Mixed liquid | | | Composition after homogenizing | | |
|---|---|---|---|---|---|---|
| | D50 µm | 3.6 µ_{↑} | 1.3µ_{↑} | D50 µm | 3.6 µ_{↑} | 1.3 µ_{↑} |
| Example 3-1 | 1.4 µm | 0% | 70.8% | 0.4 µm | 0% | 0.1% |
| Example 3-2 | 1.3 µm | 0% | 61.1% | 0.6 µm | 0% | 1.5% |
| Example 3-3 | 1.9 µm | 1.3% | 93.8% | 0.1 µm | 0% | 0% |
| Example 3-4 | 1.8 µm | 0.2% | 94.9% | 0.1 µm | 0% | 0% |
| Example 3-5 | 3.8 µm | 62.1% | 100% | 0.4 µm | 0% | 1.0% |
| Example 3-6 | 2.1 µm | 1.5% | 99.5% | 1.0 µm | 0.7% | 23.4% |
| Example 3-7 | 0.7 µm | 0% | 6.7% | 0.3 µm | 0% | 0% |

From Examples 3-1 to 3-7 containing gum ghatti and a certain amount of saponin based on 100 parts by mass of gum ghatti, emulsion compositions having a median size of not more than 4 µm were obtained only by mixing the oil phase and the aqueous phase. By further homogenizing, the emulsion composition having smaller median size was prepared. From this results, emulsion particles having excellent emulsion stability can be prepared by containing gum ghatti and a certain amount of saponin to gum ghatti.

### Experimental Example 4: Method for producing emulsion compositions (emulsion fragrance compositions)

The emulsion compositions were produced according to the formulation shown in Table 9.

The materials of the oil phase shown in Table 9 were heated to 50°C to prepare an oil phase. The oil phase was added to an aqueous phase (1), stirred at 3,000 rpm for 3 minutes to prepare a mixed liquid. An aqueous phase (2) was added to the mixed liquid, and stirred at 3,000 rpm for 1 minute. Next, the mixture was homogenized on a high-pressure homogenizer (Homogenizer 15MR-8TA, manufactured by MANTON-GAULIN) (condition: 560 kg/cm², four times).

**[Table 9]**

| Formulation of emulsion composition, % by mass | | Example 4 | Comparative Examples 4 |
|---|---|---|---|
| Oil phase | Lemon essential oil | 3 | 3 |
| | Tocopherol | 0.1 | 0.1 |
| | Medium-chain fatty acid triglyceride | 1.9 | 1.9 |
| | Total of oil phase | 5 | 5 |
| Aqueous phase (1) | 20% aqueous gum ghatti solution | 20 | 20 |
| | Quillajanin C-50-MT (G), the content of the saponin in the emulsion composition in parentheses | 0.3 (0.0375) | - |
| | Ion-exchanged water | 21.2 | 21.5 |
| Aqueous phase (2) | First lactic acid (50% aqueous solution) | 0.5 | 0.5 |
| | Glycerin | 53 | 53 |

An emulsion stability test was performed for the emulsion composition (the composition after homogenizing). The test items "D50 µm", "1.3 µm_{↑}", and "0.1% E (720 nm)" were evaluated in the emulsion stability test. Results are shown in Table 10.

**[Table 10]**

| Emulsion composition | Composition after homogenizing | | |
|---|---|---|---|
| | D50 µm | 1.3 µ_{↑} | 0.1%E(720nm) |
| Example 4 | 0.5 µm | 1.6% | 0.051 |
| Comparative Examples 4 | 0.9 µm | 15.5% | 0.114 |

Table 10 showed that the emulsion particle size of the emulsion composition was decreased by including a certain amount of saponin in addition to gum ghatti, and thereby the turbidity of the emulsion composition was decreased. Note that the acetone insolubles in the emulsion composition of Example 4 were less than 0.025% by mass.

### Experimental Example 5 Coloring food and beverage with emulsion composition (crab-flavor steamed fish paste)

Using the emulsion composition of Example 1-2, crab-flavor steamed fish paste was colored.

To 60 parts by mass of a minced fish (minced white-fleshed fish (manufactured by DAIREI CO., LTD.) were added 30 parts by mass of ice-cold water, and 10 parts by mass of the diluted solution of the emulsion composition of Example 1-2, and mixed so as to homogenize with a mortar to prepare a coloring mixture. In the coloring mixture, the content of the emulsion composition of Example 1-2 is 2.1% by mass. The coloring mixture was applied on the top surface of the crab-flavor steamed fish paste (non-colored part), steamed at 95°C for 15 minutes, and then packaged with a vacuum pack. Next, heat sterilization (boil sterilization) was performed at 90°C for 1 minute.

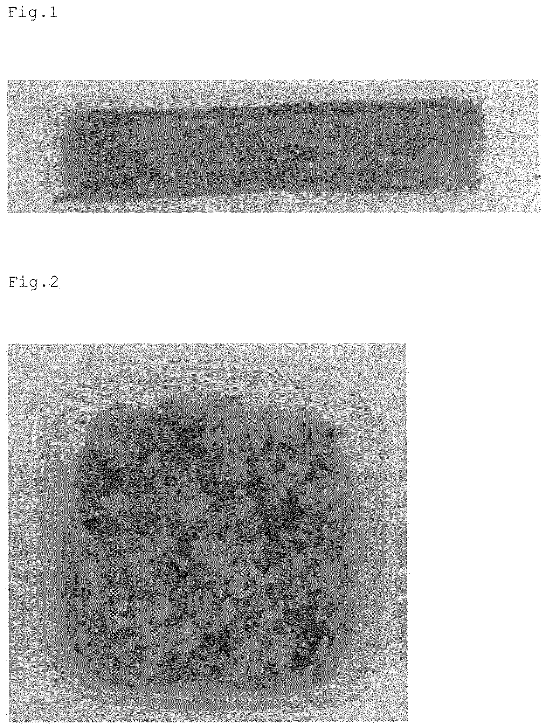

The photograph of the crab-flavor steamed fish paste after coloring is shown in Fig. 1. As shown in Fig. 1, the crab-flavor steamed fish paste having homogeneously colored into the color tone of interest without the color unevenness was obtained.

### Experimental Example 6 Coloring food and beverage with emulsion composition (Bibimbap fried rice)

Using the emulsion composition of Example 1-2, a colored Bibimbap fried rice was prepared.

According to the formulation in Table 11, water, and the emulsion composition of Example 1-2 were mixed to polished rice washed with water, and then cooked. Next, the Bibimbap fried rice was prepared according to the formulation of Table 12.

**[Table 11]**

| Cooked rice part, formulation, unit: g | |
|---|---|
| Polished rice | 300 |
| Water | 340 |
| Emulsion composition of Example 1-2 | 0.45 |
| Total | 640.45 |

**[Table 12]**

| Bibimbap fried rice, formulation, unit: g | |
|---|---|
| Cooked rice (described above) | 670 |
| Filling (including Osmund, sprout, and the like) | 100 |
| Fried egg | 70 |
| Roast pig | 40 |
| Onion | 40 |
| Seasoning sauce for fried rice | 14.2 |
| Dietary salt | 2.0 |
| Pepper | 0.2 |
| Vegetable oil | 10 |
| Total | 946.4 |

The photograph of the Bibimbap fried rice after coloring is shown in Fig. 2. The step of cooking rice is a severe producing step in which heating and convection are occurred. However, according to the composition of the present invention (the emulsion composition of Example 1-2), Bibimbap fried rice with homogeneously colored rice could be prepared (Fig. 2).

### Experimental Example 7 Coloring food and beverage with emulsion composition (Kimchi)

Using the emulsion composition of Example 1-2, a colored Kimchi was prepared.

0.2 g of the emulsion composition of Example 1-2 was added to 68 g of Chinese cabbage pickles , and mixed to prepare a colored Kimchi.

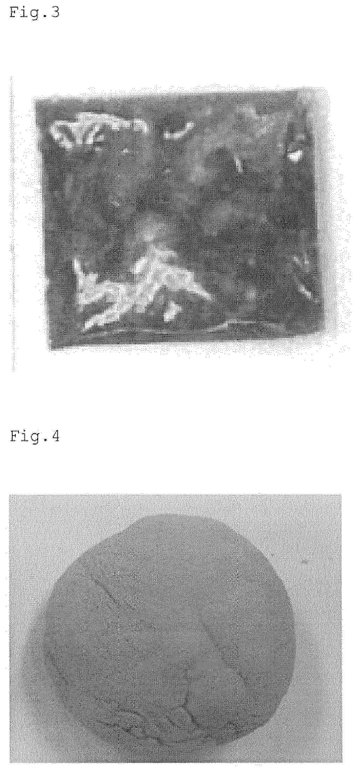

The photograph of the Kimchi after coloring is shown in Fig. 3. As shown in Fig. 3, the pickles have high salt concentration, and Kimchi having homogeneously colored into the color tone of interest without the color unevenness and the aggregation of the pigment was prepared.

### Experimental Example 8 Coloring food and beverage with emulsion composition (Chinese steamed bun)

According to the formulation of Table 13 and using the emulsion composition of Example 1-2, a colored Chinese steamed bun was prepared. A dry yeast was added to a hot water obtained by dissolving sugar, and pre-fermented for 5 to 10 minutes. The yeast liquid was gradually added to a weak flour, mixed it so as to obtain a flaky paste. After adding the emulsion composition of Example 1-2, the dough was mixed so that a gloss was occurred at the surface, and then fermented for 1 to 2 hours.

The photograph of the Chinese steamed bun after fermentation is shown in Fig. 4. As shown in Fig. 4, Chinese steamed bun having homogeneously colored into the color tone of interest without the color unevenness was prepared.

### Experimental Example 9: Method for producing emulsion compositions (emulsion pigment compositions)

The emulsion compositions were produced according to the formulation shown in each Table.

The materials of the oil phase shown in Table 13 were mixed and heated to 80°C to prepare an oil phase. The oil phase was added to an aqueous phase (1), stirred at 3,000 rpm for 3 minutes to prepare a mixed liquid. An aqueous phase (2) was added to the mixed liquid, and stirred at 3,000 rpm for 1 minute. Next, the mixture was homogenized on a high-pressure homogenizer (Homogenizer 15MR-8TA, manufactured by MANTON-GAULIN) (condition: 500 kg/cm², four times).

The acetone insolubles content in the emulsion composition is 1.8% by mass. The pH of the emulsion composition prepared in Experimental Example 9 (Examples and Comparative Example) was within a range of 2.5 to 3.5.

**[Table 13]**

| Formulation of emulsion composition, % by mass | | Example 9-1 | Example 9-2 | Comparative Examples 9-1 |
|---|---|---|---|---|
| Oil phase | Carotenoid pigment (Paprika pigment (2) color value 2,240) | 25.25 | 25.25 | 25.25 |
| | Tocopherol | 1 | 1 | 1 |
| | Medium-chain fatty acid triglyceride | 3.75 | 3.75 | 3.75 |
| | Total of oil phase | 30 | 30 | 30 |
| Aqueous phase (1) | 25% aqueous small molecule gum ghatti solution | 28 | 28 | 28 |
| | Quillajanin C-50-MT (G), the content of the saponin in parentheses | 8 (1) | 16 (2) | |
| | Ascorbic acid | 0.5 | 0.5 | 0.5 |
| | Glycerin | 0.5 | 0 | 8.5 |
| | Ion-exchanged water | 17.5 | 10 | 17.5 |
| Aqueous phase (2) | 50% aqueous lactic acid solution | 0.5 | 0.5 | 0.5 |
| | Glycerin | 15 | 15 | 15 |
| Total | | 100 | 100 | 100 |

An emulsion stability test was performed for the emulsion composition (the mixed liquid of the oil phase, the aqueous phase (1) and the aqueous phase (2), and the composition after homogenizing). The test items "D50 µm", "3.6 µm_{↑}", and "1.3 µm_{↑}" were evaluated in the emulsion stability test. Results are shown in Table 14.

**[Table 14]**

| Emulsion composition | Mixed liquid | | | Composition after homogenizing | | |
|---|---|---|---|---|---|---|
| | D50 µm | 3.6 µ_{↑} | 1.3 µ_{↑} | D50 µm | 3.6 µ_{↑} | 1.3 µ_{↑} |
| Example 9-1 | 1.9 µm | 0.2% | 96.8% | 0.4 µm | 0% | 0.6% |
| Example 9-2 | 1.1 µm | 0% | 32.1% | 0.2 µm | 0% | 0% |
| Comparative Examples 9-1 | Separated | | | homogenizing impossible | | |

For the composition of Comparative Example 9-1 using small molecule gum ghatti only, the oil was separated during the preparation of the mixed liquid. Next, the composition was continued stirring. Since the oil phase and the aqueous phase could not be mixed thoroughly, the composition was determined that it was impossible to be emulsified.

On the other hand, from Examples 9-1 and 9-2 containing gum ghatti and a certain amount of saponin based on 100 parts by mass of small molecule gum ghatti, emulsion compositions having a median size of not more than 2 µm were obtained only by mixing the oil phase and the aqueous phase. By further homogenizing, the emulsion composition having smaller median size was prepared. From these results, emulsion particles having excellent emulsion stability can be prepared by containing small molecule gum ghatti and a certain amount of saponin to small molecule gum ghatti.

### Experimental Example 10: pulverizing test

According to the formulation shown in Table 15, an emulsion composition was produced and the pulverizing test was performed.

### (Method for producing the emulsion composition having the powder form)

The materials of the oil phase were mixed and heated to 80°C to prepare an oil phase. The oil phase was added to an aqueous phase (1), stirred at 3,000 rpm for 3 minutes to prepare a mixed liquid. Next, the mixture was homogenized on a high-pressure homogenizer (Homogenizer 15MR-8TA, manufactured by MANTON-GAULIN) (condition: 500 kg/cm², four times). An aqueous phase (2) containing dextrin was added as a pulverizing substrate to the liquid after homogenizing, and stirred at 1,500 rpm for 1 minute. The resulting mixed liquid was spray-dried using a spray dryer (spray dryer, APV Nordic Anhydro) at an inlet temperature of 160°C and an outlet temperature of 85 to 90°C at 20,000 to 25,000 rpm to obtain an emulsion composition having a powder form.

The paprika pigment content in the emulsion composition having a powder form was 18.94% by mass (theoretical value), the content of the gum ghatti is 5.5% by mass (theoretical value), and the content of the saponin is 0.425% by mass (theoretical value).

**[Table 15]**

| Formulation of emulsion composition, parts by mass | | Example 10-1 |
|---|---|---|
| Oil phase | carotenoid pigment (paprika pigment (2), color value 2240) | 18.94 |
| | Tocopherol | 0.5 |
| | Medium-chain fatty acid triglyceride | 1.06 |
| | Total of oil phase | 20.5 |
| Aqueous phase (1) | 20% aqueous gum ghatti solution | 27.5 |
| | Quillajanin C-50-MT (G), the content of the saponin in parentheses | 3.4 (0.425) |
| | Ion-exchanged water | 23.6 |
| Aqueous phase (2) | Dextrin | 35 |
| | Ion-exchanged water | 40 |
| Total | | 150 |

An emulsion stability test was performed for the emulsion composition (the mixed liquid of the oil phase and the aqueous phase (1), and the composition after homogenizing), and the emulsion composition having a powder form. The emulsion stability test for the emulsion composition having a powder form was performed as a diluted aqueous solution with ion-exchanged water. The test items "D50 µm", "3.6 µm_{↑}", and "1.3 µm_{↑}" were evaluated in the emulsion stability test. Results are shown in Table 16.

**[Table 16]**

| Emulsion composition | D50 µm | 3.6 µ_{↑} | 1.3 µ_{↑} |
|---|---|---|---|
| Mixed liquid | 2.0 µm | 1.4% | 97.5% |
| After homogenizing | 0.8 µm | 0% | 6.2% |
| After pulverizing | 0.9 µm | 0% | 35.0% |

As shown in Table 16, it was confirmed that the emulsion composition having smaller emulsion particle size and excellent re-dispersibility after pulverizing could be prepared by including a certain amount of saponin in addition to gum ghatti.

## Claims

1. An emulsion composition comprising:
water, an oily component, gum ghatti, and saponin, wherein
a content of the saponin is from 0.05 to 30 parts by mass based on 100 parts by mass of the gum ghatti.

2. The emulsion composition according to claim 1, wherein the content of the saponin is from 0.01 to 10 parts by mass based on 100 parts by mass of the oily component.

3. The emulsion composition according to claim 1 or 2, wherein the oily component contains at least one kind selected from the group consisting of an oil-soluble pigment, an oil-soluble fragrance, an oil-soluble physiologically active substance, and an oily solvent.

4. The emulsion composition according to any one of claims 1 to 3, wherein the oily component contains a carotenoid pigment.

5. The emulsion composition according to any one of claims 1 to 3, wherein the oily component contains an oil-soluble fragrance.

6. The emulsion composition according to any one of claims 1 to 5, wherein emulsion particles in the emulsion composition have a median size of not more than 4 µm.

7. The emulsion composition according to any one of claims 1 to 6, wherein the emulsion composition has a liquid, powder, granule, or tablet form.

8. A method for producing an emulsion composition, comprising:
preparing a mixed liquid containing water, an oily component, gum ghatti, and saponin, where a content of the saponin is from 0.05 to 30 parts by mass based on 100 parts by mass of the gum ghatti; and
homogenizing the mixed liquid.

9. A method for producing a composition having a powder, granule, or tablet form, the method comprising the step of pulverizing the emulsion composition according to claim 8.

10. A method for producing a composition selected from the group consisting of a food and beverage, a cosmetic, a pharmaceutical product, a quasi-pharmaceutical product, a sanitary daily good, or an animal feed, the method comprising dissolving or dispersing the emulsion composition according to any one of claims 1 to 7, or the composition having a powder, granule, or tablet form according to claim 9 in an aqueous solvent.

11. A food and beverage, a cosmetic, a pharmaceutical product, a quasi-pharmaceutical product, a sanitary daily good, or an animal feed, comprising the emulsion composition according to any one of claims 1 to 7, or the composition having a powder, granule, or tablet form according to claim 9.
